# NEUE EUROPÄISCHE PATENTSCHRIFT

(11) **EP 1 986 733 B2**
(45) Veröffentlichungstag und Bekanntmachung des Hinweises auf die Entscheidung über den Einspruch: **22.09.2021**
(45) Hinweis auf die Patenterteilung: 11.07.2012
(21) Anmeldenummer: 07722770.0
(22) Anmeldetag: 26.01.2007
(51) Int. Cl.: A61N 1/05, A61N 1/40

(54) **VORRICHTUNG MIT FLEXIBLEM MEHRSCHICHTSYSTEM ZUR KONTAKTIERUNG ODER ELEKTROSTIMULATION VON LEBENDEN GEWEBEZELLEN ODER NERVEN**
DEVICE WITH FLEXIBLE MULTILAYER SYSTEM FOR CONTACTING OR ELECTROSTIMULATION OF LIVING TISSUE CELLS OR NERVES
DISPOSITIF AVEC UN SYSTEME MULTICOUCHE FLEXIBLE POUR LA MISE EN CONTACT OU L'ELECTROSTIMULATION DE CELLULES TISSULAIRES OU DE NERFS VIVANTS

(30) Priorität: 21.02.2006 DE 102006008050
(43) Veröffentlichungstag der Anmeldung: 05.11.2008
(73) Patentinhaber: Pixium Vision SA, 75012 Paris (FR)
(72) Erfinder: TIEDTKE, Hans-Jürgen, 53227 Bonn (DE)
(74) Vertreter: Graf von Stosch, Andreas
(86) Internationale Anmeldenummer: PCT/EP2007/000685
(87) Internationale Veröffentlichungsnummer: WO 2007/101508

(56) Entgegenhaltungen:
- WO-A-95/31145
- WO-A-99/49933
- DE-A1- 19 750 043
- US-A- 4 926 879
- US-A- 4 969 468
- US-A- 5 505 201
- US-A- 5 750 926
- US-A1- 2002 108 781
- US-A1- 2004 021 022
- US-A1- 2004 082 875
- US-A1- 2004 094 841
- US-A1- 2005 085 009
- US-A1- 2005 173 809
- US-A1- 2006 003 090
- US-B1- 6 477 034
- US-B2- 6 633 081
- US-B2- 6 974 533
- Feltham A M et al: "Plantinized platinum electrodes", Chemical Reviews, vol. 71, no. 2, 1971,
- Gross M: "Micromachining of flexible neural implants with low-ohmic wire traces using electroplating", Sensors and actuators, vol. A 96, 2002, pages 105-110,
- Sieglitz: "A biohybrid system to interface peripheral nerves after traumatic lesions: design of a high channel sieve electrode", Biosensors and bioelectronics, vol. 17, 2002, pages 685-696,
- "Electrochemical guidelines for selection of protocols and electrode materials for neural stimulation" In: Robblee LS. Rose TL: "Neural Prosthesis Fundamental Studies", 1990, Englewood Cliffs, NJ pages 22-26,
- Agnew W F: "Neural Prosthesis Fundamental Studies", 1990, Englewood Cliffs, NJ pages title page-contents page,

## Beschreibung

Die vorliegende Erfindung bezieht sich allgemein auf Systeme, die der Kontaktierung von lebendem Gewebe oder Nerven dienen. Die Erfindung betrifft eine Vorrichtungen zur Kontaktierung oder Elektrostimulation von lebenden Gewebezellen oder Nerven mit einer Leiterplatte umfassend mindestens einer Kontaktstelle zur elektrischen Kontaktierung von Implantaten mit Mehrschichtsystemen mit Leiterplatten, bei denen mindestens ein Kontaktpad einer flexiblen Leiterplatte formschlüssig in der Leiterplatte verankert wird, wobei eine mechanische Verstärkung des Kontaktpads durch galvanisches Aufwachsen von Leiterbahnmaterial erzielt wird.

Es sind Vorrichtungen in Form von Implantaten zur Stimulation von lebendem Gewebe bekannt, wie z.B. Implantate für die Netzhaut (Retina) des menschlichen Auges oder das menschliche Innenohr. Solche Implantate umfassen in der Regel eine Anzahl von Stimulationselektroden, über die elektrische Stimulationsimpulse an das umgebende Gewebe bzw. die Zellen abgegeben werden, um so die Nerven zu stimulieren und damit deren Funktion wiederherzustellen bzw. zu verbessern.

Bekannte Implantate sind häufig Bestandteil von Systemen, die elektrische oder elektronische Komponenten zu diagnostischen Zwecken umfassen, wie z.B. die elektrische Messung von Körperfunktionen, Blutdruck, Blutzucker oder der Temperatur. Solche Systeme können auch eine Glukose-Sensorik, Ultraschall-Sensorik, Komponenten zur Bildaufnahme oder zur Tonaufnahme und insbesondere Komponenten zu aktorischen Zwecken enthalten. Bei solchen Systemen kann es sich auch um Stimulations-Systeme handeln, die Komponenten zu aktorischen Zwecken enthalten, wie z.B. zur Elektrostimulation, Defibrillation, Schallaussendung oder Ultraschallaussendung. Solche Systeme umfassen in der Regel ein Substrat in Form einer Leiterplatte, auf der die elektronischen Bauelemente angeordnet sind, mit elektrischen Kontakten, die im direkten oder indirekten Kontakt zum Körpergewebe stehen, wie z.B. Nervengewebe und Muskelgewebe oder zu Körperflüssigkeiten, wie z.B. Blut.

Um die Abmessungen der elektrischen oder elektronischen Komponenten möglichst klein zu halten, werden neben keramischen Substraten zunehmend auch flexible Leiterplatten aus Kunststoffen z.B. Polyimid, Parylene eingesetzt. Solche flexiblen Leiterplatten lassen sich mit Hilfe von etablierten Prozessen zur Herstellung von Mikrochips in sehr feinen Abmessungen mit einer Schichtdicke der Leiterbahn von einigen Nanometer bis zu einigen Hundert Nanometer und einer Leiterbahnbreite von beispielsweise wenigen Mikrometer strukturieren.

Eine solche flexible Leiterplatte besteht in der Regel aus einer oder mehreren isolierenden Schichten beispielsweise aus Polyimid, Parylene oder anderen Kunststoffen, Isolatoren oder flexiblen Halbleitern, auf denen Leiterbahnen, Kontaktflächen oder gegebenenfalls Durchkontaktierungen zwischen mehreren Leiterbahnebenen eingerichtet sind. Zur elektrischen Kontaktierung der Leiterbahnen sind entsprechende Kontaktstellen bzw. Kontaktpads vorgesehen, über die beispielsweise elektrische Leitungen und/oder Bauelemente angeschlossen werden können, um die elektronischen Komponenten auf der Leiterplatte mit externen Komponenten des Stimulations-Systems zu verbinden.

Bei diesen flexiblen Leiterplatten wie aus DE-A-19750043 oder WO-A-99/49934 bekannt besteht jedoch das Problem, dass die dünnen Leiterbahnen insbesondere an ihren Kontaktstellen mechanisch äußerst empfindlich sind, was zum Verlust des elektrischen Kontakts zu den Kontaktstellen und den Leiterbahnen führen kann. Ein weiteres Problem dieser Kontaktstellen bzw. Kontaktpads besteht in der Verankerung innerhalb der sehr dünnen, flexiblen Leiterplatte für das Implantat. Die Leiterbahn hat in den Regel eine Dicke von einigen Nanometer bis zu einigen Hundert Nanometer und die Isolierschicht von wenigen Mikrometer. Kritisch ist dabei, dass die Kontaktstelle in der Leiterbahn nur unzureichend mechanisch verankert ist. Insbesondere durch mechanische Belastungen beim Herstellen, Bestücken oder Bearbeiten der Leiterplatten für ein Implantat aufgrund von mechanischer Druckkraft, Zugkraft, Scherkraft, Biegebeanspruchung, Dehnung, Vibration durch Ultraschall usw. kann ein Kontaktpad aus der flexiblen Leiterplatte leicht herausgelöst werden.

Es ist deshalb eine Aufgabe der vorliegenden Erfindung eine Leiterplatte für ein Implantat mit verbesserten Eigenschaften bei der elektrischen Kontaktierung über die Kontaktstellen der Leiterbahnen auf der Leiterplatte zu schaffen.

Diese Aufgabe wird durch die erfindungsgemäße Vorrichtung mit den Merkmalen gemäß Anspruch 1 gelöst. Vorteilhafte Weiterbildungen der Erfindung sind jeweils in den Unteransprüchen angegeben.

Gemäß der vorliegenden Erfindung wird die oben genannte Aufgabe gelöst durch eine Vorrichtung zur Kontaktierung und/oder Elektrostimulation von lebenden Gewebezellen oder Nerven mit einer Leiterplatte, die mindestens eine elektrisch isolierende Materialschicht umfasst, auf der eine Leiterbahn-Schicht mit mindestens einer Leiterbahn angeordnet ist. Zur elektrischen Kontaktierung der Leiterbahn ist mindestens eine Kontaktstelle vorgesehen. Über der Leiterbahn ist mindestens eine zusätzliche Materialschicht angeordnet, durch die sich die Kontaktstelle hindurch erstreckt. Auf diese Weise ist die Leiterbahn über die Kontaktstelle durch die zusätzliche Materialschicht hindurch von außerhalb der Leiterplatte elektrisch kontaktierbar.

Mit dem erfindungsgemäßen Verfahren werden folglich die Kontaktstellen für die Leiterbahnen des Implantats, wie z.B. eine Stimulationselektrode, mittels einer oder mehrerer zusätzlicher Materialschichten verstärkt. Dabei wird beispielsweise durch einen galvanischen Prozess auf die schon vorprozessierte Kontaktstelle eine galvanisch verstärkte Schicht aufgewachsen. Alternativ können neben dem galvanischen Metallauftrag auch andere geeignete Prozesse zum Aufbringen einer oder mehrerer Materialschichten angewendet werden, wie z.B. Sputtern. Durch das nachträgliche Auftragen einer oder mehrerer zusätzlicher Materialschichten auf die vorstrukturierten Kontaktstellen der Leiterbahnen werden diese mechanisch stabiler in der Leiterplatte integriert und damit in ihrer Funktion zuverlässiger, d.h. die Kontaktstellen können zuverlässiger kontaktiert und der elektrische Kontakt besser aufrecht erhalten werden und die Kontaktstellen sind in der Stimulationselektrode besser verankert.

Nach einer bevorzugten Ausführungsform der vorliegenden Erfindung besteht die flexible Leiterplatte für ein Implantat aus mehreren Schichten eines isolierenden Materials mit dazwischen oder darunter oder darauf angeordneten Leiterbahnen, die vor dem Aufbringen weiterer Deckschichten aus Isolationsmaterial im Bereich der Kontaktstellen mechanisch verstärkt werden. Das Auftragen einer oder mehrerer mechanisch verstärkender Schichten auf der Leiterplatte erfolgt erfindungsgemäß durch galvanische Verfahren, oder durch Sputtern.

Ebenfalls offenbart wird ein Verfahren zum Herstellen einer Leiterplatte in Form eines Mehrschichtsystems für eine Vorrichtung zur Kontaktierung und/oder Elektrostimulation von lebenden Gewebezellen oder Nerven umfassend die folgenden Schritte:
- Erzeugen einer ersten isolierenden Materialschicht,
- Erzeugen einer Leiterbahn auf der ersten isolierenden Materialschicht,
- Erzeugen einer zweiten isolierenden Materialschicht,
- Erzeugen von mindestens einem Fenster in der zweiten isolierenden Materialschicht,
- Auffüllen des Fensters in der zweiten isolierenden Materialschicht mit einem elektrisch leitenden Material zum Erzeugen einer Kontaktstelle, die einen elektrischen Kontakt mit der Leiterbahn aufweist,
- Erzeugen einer zusätzlichen isolierenden Materialschicht auf der zweiten isolierenden Materialschicht,
- Erzeugen von mindestens einem Fenster in der zusätzlichen isolierenden Materialschicht, wobei das Fenster in der zusätzlichen isolierenden Materialschicht geringere laterale Abmessungen aufweist als das Fenster in der zweiten isolierenden Materialschicht,
- Auffüllen des Fensters in der zusätzlichen isolierenden Materialschicht mit einem elektrisch leitenden Material zum Erzeugen eines Kontaktpads, das einen elektrischen Kontakt mit der Kontaktstelle aufweist.

Die Herstellung der feinen Strukturen einer für den Einsatz in einem Implantat geeigneten flexiblen Leiterplatte erfolgt in der Regel mit Hilfe von lithographischen Verfahren, die bereits für die Herstellung von Halbleitern aus Silizium-Wafern etabliert sind.

Mit diesem Verfahren lassen sich galvanisch verstärkte und formschlüssig verankerte Kontaktpads mit formschlüssiger Verankerung in flexiblen Leiterplatten erzeugen, die in Vorrichtungen mit flexiblem Mehrschichtsystem zur Kontaktierung oder Elektrostimulation von lebenden Gewebezellen oder Nerven verwendbar sind. Die Kontaktpads können wahlweise auf der Oberseite und/oder auf der Unterseite der Leiterplatte des flexiblen Mehrschichtsystems angebracht werden. Anstelle oder zusammen mit den Kontaktpads können auch Elektroden zur Elektrostimulation von lebendem Gewebe oder Nerven in der flexiblen Leiterplatte eingerichtet werden. Über Durchkontaktierungen durch die isolierenden Materialschichten der flexiblen Leiterplatte lassen sich elektrische Kontakte von den Kontaktpads bzw. von den Kontaktstellen zu den inneren Metall-Leiterbahnen des Mehrschichtsystems herstellen.

Eine weitere Besonderheit des Verfahrens zur Herstellung einer zur Verwendung in einem Implantat geeigneten Leiterplatte nach der vorliegenden Erfindung besteht darin, dass einerseits ein galvanischer Herstellungsprozess eingesetzt wird, um mechanische Verstärkungen der Kontaktpads bzw. an den Kontaktstellen zu erzielen, und dass diese galvanisch verstärkten Kontaktstellen durch innere Galvanikschichten formschlüssig in der Leiterplatte verankert werden, das heißt dass die innere Galvanikschicht des betreffenden Kontaktpads in ihren lateralen Abmessungen größer ist als die äußere Galvanikschicht des Kontaktpads, so dass eine gute mechanische Verankerung gegenüber von außen einwirkenden mechanischen Kräften gegeben ist. Diese gute mechanische Verankerung des bzw. der Kontaktpads in der Leiterplatte ist eine wichtige Voraussetzung für anschließend durchzuführende mechanische Verbindungstechniken, wie z.B. Ultraschall-Bonden, Leitkleben, Thermokompressionsbonden, Flip-Chip-Bonden und andere elektrische Verbindungsverfahren.

Eine gute mechanische Verankerung der Kontaktpads in der Leiterplatte für ein Implantat ist auch eine Voraussetzung zur Herstellung mechanisch robuster Elektroden auf der flexiblen Leiterplatte, was z.B. in den Neuroprothetik von großer Bedeutung ist. Solche Elektroden können insbesondere für elektrostimulatorische Zwecke verwendet werden, z.B. in einem Retina-Implantat, einem Cochlear-Implantat, für Himstamm-Stimulatoren, Tiefenhimstimulationen, Rückenmark-Stimulatoren oder andere Stimulatoren. Andererseits besteht auch die Möglichkeit, mit dem beschriebenen Verfahren Ableitelektroden zu realisieren, die z.B. für elektrophysiologische Messungen der Nervenaktivität oder der Impedanz von biologischen oder chemischen Systemen verwendet werden können.

Weitere Einzelheiten, bevorzugte Ausführungsformen und Vorteile der vorliegenden Erfindung ergeben sich aus der nachfolgenden Beschreibung unter Bezugnahme auf die beigefügten Zeichnungen. Es zeigen:
- Figur 1: eine schematische Darstellung vom Aufbau einer Leiterplatte gemäß einer ersten bevorzugten Ausführungsform der vorliegenden Erfindung zur Verwendung in einer Vorrichtung zur Kontaktierung oder Elektrostimulation von lebenden Gewebezellen oder Nerven;
- Figur 2a: eine schematische Darstellung vom Aufbau einer Leiterplatte gemäß einer zweiten bevorzugten Ausführungsform der vorliegenden Erfindung zur Verwendung in einer Vorrichtung zur Kontaktierung oder Elektrostimulation von lebenden Gewebezellen oder Nerven;
- Figur 2b: eine schematische Schnittdarstellung der in Figur 2a gezeigten Ausführungsform der erfindungsgemäßen Leiterplatte.
- Figur 3a: eine schematische Darstellung vom Aufbau einer Leiterplatte gemäß einer dritten bevorzugten Ausführungsform der vorliegenden Erfindung zur Verwendung in einer Vorrichtung zur Kontaktierung oder Elektrostimulation von lebenden Gewebezellen oder Nerven; und
- Figur 3b: zeigt eine schematische Schnittdarstellung der in Figur 3a gezeigten Ausführungsform der erfindungsgemäßen Leiterplatte.

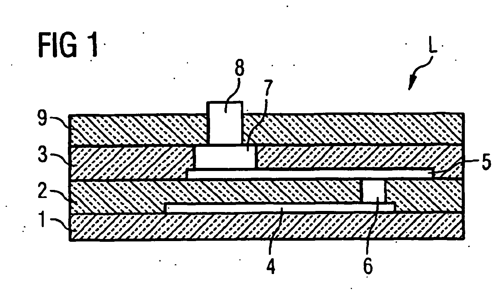

In Figur 1 ist eine schematische Darstellung vom Aufbau einer Leiterplatte L gemäß einer ersten bevorzugten Ausführungsform der vorliegenden Erfindung zur Verwendung in einer Vorrichtung zur Kontaktierung oder Elektrostimulation von lebenden Gewebezellen oder Nerven.

Die in Figur 1 dargestellte Leiterplatte L umfasst drei Materialschichten 1, 2, 3 aus einem elektrisch isolierenden Material, wie z.B. aus Polyimid, Parylene oder einem anderen Isolator. Auf diesen isolierenden Schichten 1, 2, 3 sind eine untere Leiterbahn 4 und eine obere Leiterbahn 5 ausgebildet. Die Leiterbahnen 4 und 5 liegen jeweils in einer entsprechenden Leiterbahnebene, wobei sich die Leiterbahnebene der Leiterbahn 4 zwischen der isolierenden Materialschicht 1 und der isolierenden Materialschicht 2 befindet und die Leiterbahnebene der Leiterbahn 5 zwischen der isolierenden Materialschicht 2 und der isolierenden Materialschicht 3 liegt. Bei der in Figur 1 dargestellten Ausführungsform der erfindungsgemäßen Leiterplatte L sind die Leiterbahnen 4 und 5 über eine Durchkontaktierung 6 zwischen den Leiterbahnebenen elektrisch miteinander verbunden.

Die obere Leiterbahn 5 ist ferner mit einer Kontaktfläche bzw. einem Kontaktpad 7 versehen, das sich von der Leiterbahnebene der oberen Leiterbahn 5 bis zum äußeren Rand der Leiterplatte L erstreckt. Zur elektrischen Kontaktierung der Leiterbahnen sind entsprechende Kontaktstellen 7 vorgesehen, an denen externe elektrische Leitungen angeschlossen werden können, um die elektronischen Komponenten auf der Leiterplatte L mit weiteren elektrischen Komponenten eines Systems zu verbinden. Auf die Kontaktstelle 7 kann auch eine Elektrode 8 aufgebracht werden. Die Leiterbahnen 4, 5 können beispielsweise aus Titan, Kupfer, Gold, Silber, Platin, Leitkunststoff oder anderen elektrisch leitfähigen Materialien aufgebaut sein. Die erfindungsgemäße Leiterplatte L kann durch Prozesse zum Erzeugen von Mikrostrukturen hergestellt werden, wie sie z.B. aus der Produktion von Halbleiter-Bauelementen bekannt sind. Mit Hilfe solcher Prozesse können sehr feine Abmessungen der Leiterplatte L erzielt werden, wobei die Schichtdicke der Leiterbahnen 4, 5 einige Nanometer bis zu einigen Hundert Nanometer und die Schichtdicke der isolierenden Materialschichten 1, 2, 3 wenige Mikrometer betragen kann.

Um die oben genannte Aufgabe einer mechanisch stabileren und funktionell zuverlässigeren Anordnung der Kontaktstellen 7 bzw. Elektrodenpads 8 in der Leiterplatte L zu erfüllen, werden nach der vorliegenden Erfindung die Kontaktstellen 7 für die Leiterbahnen 5 mittels einer oder mehrerer zusätzlicher Materialschichten 9 nachträglich verstärkt. Bei der in Figur 1 dargestellten ersten Ausführungsform der erfindungsgemäßen Leiterplatte L ist auf der Kontaktstelle 7 der Leiterplatte L eine Schicht 9 aus elektrisch isolierendem Material angeordnet. Durch die Anordnung einer oder mehrerer zusätzlicher Materialschichten 9 auf der Kontaktstelle 7 der Leiterbahn 5 wird die Kontaktstelle 7 mechanisch stabiler in der Leiterplatte L verankert und kann damit zuverlässiger kontaktiert werden.

Auf der Kontaktstelle 7 ist ein Kontaktpad 8 aus einem elektrisch leitfähigen Material angeordnet, das sich durch die zusätzliche Materialschicht 9 hindurch erstreckt, über das die Kontaktstelle 7 von außen kontaktiert werden kann. Dies geschieht vorzugsweise durch Sputtern, galvanisches Aufwachsen, Aufkleben mit Hilfe eines leitfähigen Klebers, durch Löten, Schweißen, Ultraschall-Bonden, Thermokompressionsbonden, Anpressen oder Anklemmen. Es können auch mehrere zusätzliche Materialschichten 9 auf der Materialschicht 3 über der Leiterbahnebene mit der oberen Leiterbahn 5 angeordnet sein. Dann ist das Kontaktpad 8 aus elektrisch leitendem Material so ausgebildet, dass es sich von der Kontaktstelle 7 durch sämtliche zusätzlichen Materialschichten 9 hindurch erstreckt, um die Kontaktstelle 7 von außerhalb der Leiterbahnen elektrisch kontaktieren zu können. Auf diese Weise können galvanisch verstärkte und formschlüssig verankerte Kontaktpads 8 oder auch Elektroden sowohl auf der Oberseite als auch auf der Unterseite der Leiterplatte L eingerichtet werden, die über jeweilige Kontaktstellen 7 einen elektrischen Kontakt zu den inneren Metall-Leiterbahnen 4 und 5 der Leiterplatte L ermöglichen.

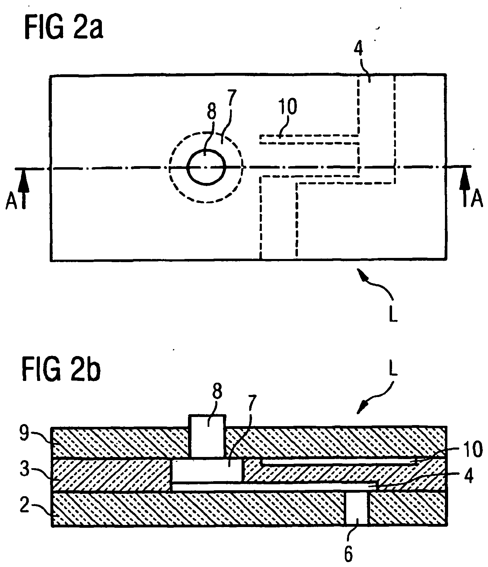

Figur 2a zeigt eine schematische Darstellung vom Aufbau einer Leiterplatte L gemäß einer zweiten bevorzugten Ausführungsform der vorliegenden Erfindung mit einer an der Oberseite der Leiterplatte L ausgebildeten Kontaktstelle 7, 8 und Figur 2b zeigt eine schematische Schnittdarstellung der in Figur 2a gezeigten Ausführungsform der erfindungsgemäßen Leiterplatte L. Die dort gezeigte Ausführungsform einer erfindungsgemäßen Leiterplatte L umfasst wiederum drei isolierende Materialschichten 2, 3, und 9, sowie zwei Leiterbahn-Schichten mit elektrisch leitenden Leiterbahnen 4 und 10. Die untere Leiterbahn 4 liegt zwischen den isolierenden Materialschichten 2 und 3, während die obere Leiterbahn 10 zwischen den isolierenden Materialschichten 3 und 9 angeordnet ist.

Die untere Leiterbahn 4 ist über eine Durchkontaktierung 6 elektrisch kontaktiert und die untere Leiterbahn 4 ist mit einer Kontaktstelle 7 ausgestattet, auf der ein Kontaktpad oder Elektrodenpad 8 angeordnet ist. Sowohl die Durchkontaktierung 6 als auch die Kontaktstelle 7 und das Kontaktpad 8 sind in Form einer Galvanikschicht ausgebildet, d.h. sie sind als galvanischer Metallauftrag durch einen galvanischen Prozess erzeugt worden. Über der oberen Leiterbahn 10 ist eine zusätzliche Isolatorschicht 9 angeordnet, die oberhalb der Kontaktstelle 7 liegt und das Kontaktpad 8 in sich einschließt. Durch den Einsatz galvanischer Prozesse wird folglich eine Verstärkung der Kontaktstelle 7 bzw. des Kontaktpads 8 erzielt und durch das Auftragen der zusätzlichen Materialschicht 9 auf der oberen Leiterbahn-Schicht gegenüber der isolierenden Materialschicht 3 wird eine formschlüssige Verankerung der Kontaktstelle 7, 8 auf der Oberseite der Leiterplatte L erzielt.

Das Kontaktpad 8 erstreckt sich von der Kontaktstelle 7 bis an den oberen Rand oder darüber hinaus der zusätzlichen Materialschicht 9, so dass die Leiterbahn 4 von außerhalb der Leiterplatte L elektrisch kontaktierbar ist. Dadurch umfasst die Kontaktstelle einen unteren Teil 7 und einen oberen Teil 8, wobei der untere Teil 7 eine größere laterale Abmessung aufweist als der obere Teil 8. Auf diese Weise ist die Kontaktstelle 7, 8 in der Leiterplatte L besser verankert und gegenüber mechanischen Verformungen und/oder Zugkräften senkrecht zu der flexiblen Leiterplatte L weniger empfindlich.

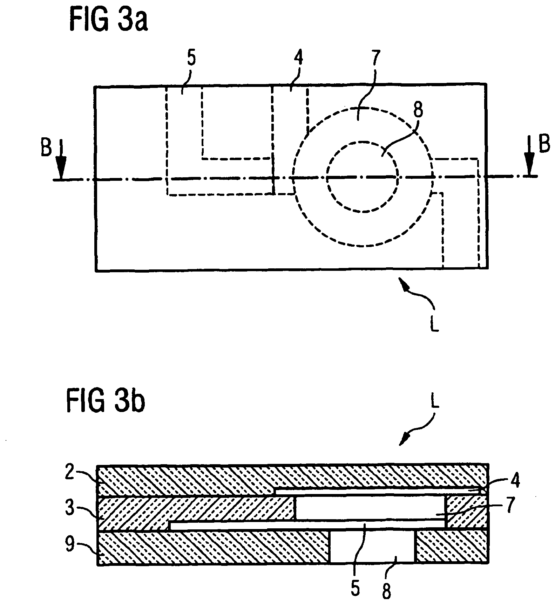

Figur 3a zeigt eine schematische Darstellung vom Aufbau einer Leiterplatte L gemäß einer dritten bevorzugten Ausführungsform der vorliegenden Erfindung mit einer an der Unterseite der Leiterplatte L ausgebildeten Kontaktstelle 7, 8 und Figur 3b zeigt eine schematische Schnittdarstellung der in Figur 3a gezeigten Ausführungsform der erfindungsgemäßen Leiterplatte L. Die dort gezeigte Ausführungsform einer erfindungsgemäßen Leiterplatte L hat zum Teil einen ähnlichen Aufbau wie die in den Figuren 2a und 2b gezeigte Ausführungsform.

Die in den Figuren 3a und 3b gezeigte Ausführungsform einer erfindungsgemäßen Leiterplatte L weist an der Unterseite eine Kontaktstelle 7, 8 auf und umfasst wiederum drei isolierende Materialschichten 2, 3, und 9, sowie zwei Leiterbahn-Schichten mit elektrisch leitenden Leiterbahnen 4 und 5. Die obere Leiterbahn 4 liegt zwischen den isolierenden Materialschichten 2 und 3, während die untere Leiterbahn 5 zwischen den isolierenden Materialschichten 3 und 9 angeordnet ist.

Die obere Leiterbahn 4 ist über eine Kontaktstelle 7 und die untere Leiterbahn 5 über ein Kontaktpad 8 elektrisch kontaktiert. Die Kontaktstelle 7 und das Kontaktpad 8 sind wiederum durch einen galvanischen Prozess erzeugt worden. Unterhalb der unteren Leiterbahn 5 ist eine zusätzliche isolierende Materialschicht 9 angeordnet, die unter der Kontaktstelle 7 liegt und das Kontaktpad 8 umschließt. Das Kontaktpad 8 erstreckt sich von der Kontaktstelle 7 bis an den unteren Rand oder darüber hinaus der zusätzlichen Materialschicht 9, so dass die Leiterbahn 4 von außerhalb der Leiterplatte L elektrisch kontaktierbar ist. Auf diese Weise besteht die Kontaktstelle wieder aus einem ersten Teil 7, der eine größere laterale Abmessung aufweist als der zweite Teil 8 und ist dadurch in der Leiterplatte L zuverlässig verankert.

### Liste der Bezugszeichen

- 1: isolierende Materialschicht bzw. Isolatorschicht
- 2: isolierende Materialschicht bzw. Isolatorschicht
- 3: isolierende Materialschicht bzw. Isolatorschicht
- 4: Leiterbahn bzw. Leiterbahnebene
- 5: Leiterbahn bzw. Leiterbahnebene
- 6: Durchkontaktierung
- 7: unterer Teil der Kontaktstelle
- 8: oberer Teil der Kontaktstelle bzw. Kontaktpad
- 9: isolierende Materialschicht bzw. Isolatorschicht
- 10: Leiterbahn bzw. Leiterbahnebene
- A: Schnittebene der Darstellung in Figur 2b
- B: Schnittebene der Darstellung in Figur 3b
- L: Leiterplatte

## Patentansprüche

1. Vorrichtung zur Kontaktierung und/oder Elektrostimulation von lebenden Gewebezellen oder Nerven mit einer Leiterplatte (L) umfassend mindestens eine Kontaktstelle (7, 8) zur elektrischen Kontaktierung, wobei die Leiterplatte (L) einen Aufbau mit folgenden Schichten umfasst:
- mindestens eine elektrisch isolierende Materialschicht (1, 2, 3),
- mindestens eine Leiterbahn-Schicht (4, 5, 10) mit einer Leiterbahn (4, 5, 10), und
- mindestens eine über der Leiterbahn (4, 5, 10) vorgesehene zusätzliche isolierende Materialschicht (9),
wobei die Leiterbahn (4, 5, 10) über die Kontaktstelle (7, 8) durch die zusätzliche Materialschicht (9) hindurch von außerhalb der Leiterplatte (L) elektrisch kontaktierbar ist, wobei die Kontaktstelle (7) eine erste Oberfläche, welche in direktem Kontakt mit einer Leiterbahn-Schicht (4, 5, 10) ist, und einen zweite, gegenüberliegende Oberfläche, welche von der mindestens einen zusätzlichen isolierenden Materialschicht bedeckt ist, umfasst,
wobei die Kontaktstelle (7) durch einen galvanischen Metallauftrag oder durch Sputtern auf die Leiterbahn (4, 5) aufgebracht ist, sodass die Kontaktstelle (7) in der Leiterbahn integriert bzw. verankert ist,
**dadurch gekennzeichnet, dass** die Kontaktstelle (7, 8) einen ersten Teil (7) aufweist, der eine grössere laterale Abmessung aufweist als ein zweiter Teil (8) der Kontaktstelle (7, 8) und wobei der erste Teil (7) der Kontaktstelle (7, 8) zumindest teilweise von der zusätzlichen Materialschicht (9) überlagert wird, und wobei der zweite Teil (8) der Kontaktstelle (7, 8) zumindest teilweise von der zusätzlichen Materialschicht (9) umschlossen ist,
und wobei die Kontaktstelle (7) zur elektrischen Kontaktierung der Leiterbahn (4, 5, 10) ein Kontaktpad oder Elektrodenpad (8) aufweist, das von ausserhalb der Leiterplatte (L) elektrisch kontaktierbar ist und wobei das Kontaktpad (8) sich zumindest bis an einen äußeren Rand der Leiterplatte (L) erstreckt oder darüber hinaus ragt.

2. Vorrichtung nach einem der vorangehenden Ansprüche, wobei mehrere zusätzliche Materialschichten (9) vorgesehen sind, durch die sich die Kontaktstelle (7, 8) von der Leiterbahn (4, 5, 10) bis zu einem äußeren Rand der Leiterplatte (L) erstreckt.

3. Vorrichtung nach einem der vorangehenden Ansprüche, wobei die Leiterbahn (4, 5, 10) auf einer der elektrisch isolierenden Materialschichten (1, 2, 3, 9) aufgebracht ist.

4. Vorrichtung nach einem der vorangehenden Ansprüche, wobei die Leiterbahn (4, 5, 10) in einer der elektrisch isolierenden Materialschichten (1, 2, 3, 9) integriert ist.

5. Vorrichtung nach einem der vorangehenden Ansprüche, wobei die Leiterbahn (4, 5, 10) zwischen zwei elektrisch isolierenden Materialschichten (1, 2, 3, 9) angeordnet ist.

6. Vorrichtung nach einem der vorangehenden Ansprüche, wobei mehrere Leiterbahn-Schichten (4, 5, 10) vorgesehen sind, die im Wesentlichen durch mindestens eine isolierende Materialschicht (1, 2, 3, 9) voneinander getrennt sind.

7. Vorrichtung nach einem der vorangehenden Ansprüche, wobei mehrere Leiterbahn-Schichten (4, 5, 10) vorgesehen sind, die über Durchkontaktierungen oder über Galvanikschichten (6, 7) elektrisch miteinander verbunden sind.

8. Vorrichtung nach einem der Ansprüche 6 oder 7, wobei die Leiterbahn-Schichten (4, 5, 10) parallel zueinander angeordnet sind.

9. Vorrichtung nach einem der vorangehenden Ansprüche, wobei die Dicke der Leiterbahnschichten (4, 5, 10) im Bereich zwischen einigen Nanometer bis zu einigen Hundert Nanometer und die Schichtdicke der isolierenden Materialschichten (1, 2, 3, 9) im Bereich weniger Mikrometer liegt.

10. Vorrichtung nach einem der vorangehenden Ansprüche, wobei die Leiterbahn-Schichten (4, 5, 10) und die isolierenden Materialschichten (1, 2, 3, 9) parallel zueinander angeordnet sind.

11. Vorrichtung nach einem der vorangehenden Ansprüche, wobei die Kontaktstelle (7, 8) zur elektrischen Kontaktierung der Leiterbahn (4, 5, 10) auf der Oberseite und/oder auf der Unterseite der Leiterplatte (L) angeordnet ist.

12. Vorrichtung nach einem der vorhergehenden Ansprüche, wobei die Kontaktstelle (7) und/oder das Kontaktpad (8) in der zusätzlichen Materialschicht (9) integriert ist.

## Claims

1. Device for contacting and/or electrostimulation of living tissue cells or nerves with a circuit board (L) comprising at least one contact point (7, 8) for electrical contacting, the circuit board (L) comprising a structure having the following layers:
- at least one electrically insulating material layer (1, 2, 3),
- at least one conductor-track layer (4, 5, 10) having a conductor track (4, 5, 10), and
- at least one additional insulating material layer (9) provided about the conductor track (4, 5, 10),
wherein the conductor track (4, 5, 10) is electrically contactable from outside of the circuit board (L) through the additional material layer (9) via the contact point (7, 8),
wherein the contact point (7) comprises a first surface which is in direct contact with a conductor-track layer (4, 5, 10), and an opposite second surface which is covered by the at least one additional insulating material layer,
wherein the contact point (7) is applied onto the conductor-track (4,5) by galvanic application of metal or by sputtering, so that the contact point (7) is integrated or anchored within the conductor-track
**characterized in that**
the contact point (7, 8) comprises a first part (7) which exhibits a larger lateral dimension than a second part (8) of the contact point (7, 8) and
that the first part (7) of the contact point (7, 8) is at least partially overlapped by the additional material layer (9), and wherein the second part (8) of the contact point (7, 8) is at least partially surrounded by the additional material layer (9),
and
that the contact point (7) for electrically contacting the conductor track (4,5,10) exhibits a contact pad or electrode pad (8) which is electrically contactable from outside of the circuit board (L) and wherein the contact pad (8) extends at least as far as an outer edge of the circuit board (L) or projects beyond it.

2. Device according to any one of the preceding claims, wherein multiple additional material layers (9) are provided, through which the contact point (7, 8) extends from the conductor track (4, 5, 10) as far as an outer edge of the circuit board (L).

3. Device according to any one of the preceding claims, wherein the conductor track (4, 5, 10) is applied on one of the electrically insulating material layers (1, 2, 3, 9).

4. Device according to any one of the preceding claims, wherein the conductor track (4, 5, 10) is integrated within one of the electrically insulating material layers (1, 2, 3, 9).

5. Device according to any one of the preceding claims, wherein the conductor track (4, 5, 10) is arranged between two electrically insulating material layers (1, 2, 3, 9).

6. Device according to any one of the preceding claims, wherein several conductor-track layers (4, 5, 10) are provided which are substantially separated from one another by at least one insulating material layer (1, 2, 3, 9).

7. Device according to any one of the preceding claims, wherein several conductor track layers (4, 5, 10) are provided which are electrically connected to one another via through-connections or via galvanic layers (6, 7).

8. Device according to any one of claims 6 or 7, wherein the conductor-track layers (4, 5, 10) are arranged parallel to one another.

9. Device according to any one of the preceding claims, wherein the thickness of the conductor-track layers (4, 5, 10) lies within the range between a few nanometres up to a few hundred nanometres and the layer thickness of the insulating material layers (1, 2, 3, 9) lies within the range of a few micrometres.

10. Device according to any one of the preceding claims, wherein the conductor-track layers (4, 5, 10) and the insulating material layers (1, 2, 3, 9) are arranged parallel to one another.

11. Device according to any one of the preceding claims, wherein the contact point (7, 8) for electrically contacting the conductor track (4, 5, 10) is arranged on the upper side and/or on the underside of the circuit board (L).

12. Device according to any one of the preceding claims, wherein the contact point (7) and/or the contact pad (8) is/are integrated within the additional material layer (9).

## Revendications

1. Dispositif pour la mise en contact et/ou l'électrostimulation de cellules tissulaires ou de nerfs vivants avec une plaquette de circuits imprimés (L) comportant au moins un point de contact (7, 8) en vue de la mise en contact électrique, sachant que la plaquette de circuits imprimés (L) comporte une construction avec les couches suivantes :
- au moins une couche de matière isolante électriquement (1, 2, 3)
- au moins une couche de piste conductrice (4, 5, 10) avec une piste conductrice (4, 5, 10), et
- au moins une couche de matière isolante supplémentaire (9) prévue sur la piste conductrice (4, 5, 10),
sachant que la piste conductrice (4, 5, 10) peut être mise en contact électriquement par l'intermédiaire du point de contact (7, 8) au travers de la couche de matière supplémentaire (9) depuis l'extérieur de la plaquette de circuits imprimés (L), sachant que le point de contact (7) comporte une première surface, qui est en contact direct avec une couche de piste conductrice (4, 5, 10), et une seconde surface opposée, qui est recouverte par la au moins une couche de matière isolante supplémentaire, sachant que le point de contact (7) est appliqué sur la piste conductrice (4, 5) grâce à un dépôt métallique galvanique ou grâce à une opération de pulvérisation, de manière à ce que le point de contact (7) soit intégré ou ancré dans la piste conductrice, **caractérisé en ce que** le point de contact (7, 8) présente une première partie (7) qui présente une dimension latérale plus grande que celle d'une seconde partie (8) du point de contact (7, 8), et
sachant que la première partie (7) du point de contact (7, 8) est recouverte au moins partiellement par la couche de matière supplémentaire (9), et sachant que la seconde partie (8) du point de contact (7, 8) est au moins partiellement entourée de la couche de matière supplémentaire (9),
et sachant que le point de contact (7) en vue de la mise en contact électrique de la piste conductrice (4, 5, 10) présente un tampon de contact ou un tampon d'électrode (8), qui peut être mis en contact électriquement à partir de l'extérieur de la plaquette de circuits imprimés (L) et sachant que le tampon de contact (8) se prolonge tout au moins jusqu'à un bord externe de la plaquette de circuits imprimés (L) ou fait saillie hors de celle-ci.

2. Dispositif selon l'une quelconque des revendications précédentes, sachant que plusieurs couches de matière supplémentaires (9) sont prévues à travers lesquelles le point de contact (7, 8) se prolonge depuis la piste conductrice (4, 5, 10) jusqu'à un bord externe de la plaquette de circuits imprimés (L).

3. Dispositif selon l'une quelconque des revendications précédentes, sachant que la piste conductrice (4, 5, 10) est appliquée sur une des couches de matière isolantes électriquement (1, 2, 3, 9).

4. Dispositif selon l'une quelconque des revendications précédentes, sachant que la piste conductrice (4, 5, 10) est intégrée dans une des couches de matière isolantes électriquement (1, 2, 3, 9).

5. Dispositif selon l'une quelconque des revendications précédentes, sachant que la piste conductrice (4, 5, 10) est disposée entre deux couches de matière isolantes électriquement (1, 2, 3, 9).

6. Dispositif selon l'une quelconque des revendications précédentes, sachant que plusieurs couches de piste conductrice (4, 5, 10) sont prévues, qui sont essentiellement séparées les unes des autres par au moins une couche de matière isolante (1, 2, 3, 9).

7. Dispositif selon l'une quelconque des revendications précédentes, sachant que plusieurs couches de piste conductrice (4, 5, 10) sont prévues, qui sont reliées électriquement les unes aux autres par des connexions transversales ou par des couches galvaniques (6, 7).

8. Dispositif selon l'une quelconque des revendications 6 ou 7, sachant que les couches de piste conductrice (4, 5, 10) sont disposées parallèlement les unes par rapport aux autres.

9. Dispositif selon l'une quelconque des revendications précédentes, sachant que l'épaisseur des couches de piste conductrice (4, 5, 10) se situe dans la plage comprise entre quelques nanomètres et quelques centaines de nanomètres et que l'épaisseur de couche des couches de matière isolantes (1, 2, 3, 9) se situe dans la plage de quelques micromètres.

10. Dispositif selon l'une quelconque des revendications précédentes, sachant que les couches de piste conductrice (4, 5, 10) et les couches de matière isolantes (1, 2, 3, 9) sont disposées parallèlement les unes par rapport aux autres.

11. Dispositif selon l'une quelconque des revendications précédentes, sachant que le point de contact (7, 8) pour la mise en contact électrique de la piste conductrice (4, 5, 10) est disposé sur le côté supérieur et/ou sur le côté inférieur de la plaquette de circuits imprimés (L).

12. Dispositif selon l'une quelconque des revendications précédentes, sachant que le point de contact (7) et/ou le tampon de contact (8) est/sont intégré(s) dans la couche de matière supplémentaire (9).
